# EUROPÄISCHE PATENTSCHRIFT

(11) **EP 1 940 349 B1**
(45) Veröffentlichungstag und Bekanntmachung des Hinweises auf die Patenterteilung: **14.02.2018**
(21) Anmeldenummer: 06807028.3
(22) Anmeldetag: 06.10.2006
(51) Int. Cl.: A61K 31/536, A61K 9/10, A61K 31/439, A61K 9/08, A61P 11/00

(54) **AEROSOLFORMULIERUNG FÜR DIE INHALATION VON BETAAGONISTEN**
AEROSOL FORMULATION FOR THE INHALATION OF BETA AGONISTS
FORMULATION D'AEROSOL PERMETTANT L'INHALATION DE BETA-AGONISTES

(30) Priorität: 10.10.2005 EP 05109376
(43) Veröffentlichungstag der Anmeldung: 09.07.2008
(73) Patentinhaber: Boehringer Ingelheim International GmbH, 55216 Ingelheim am Rhein (DE); Boehringer Ingelheim Pharma GmbH & Co. KG, 55216 Ingelheim am Rhein (DE)
(72) Erfinder: NIKLAUS-HUMKE, Barbara, 55432 Damscheid (DE); NOWAK, Michael, 65396 Walluf (DE); RADAU, Kirsten, 55218 Ingelheim am Rhein (DE)
(74) Vertreter: Simon, Elke Anna Maria
(86) Internationale Anmeldenummer: PCT/EP2006/067126
(87) Internationale Veröffentlichungsnummer: WO 2007/042468

(56) Entgegenhaltungen:
- EP-A1- 0 073 505
- WO-A-01/83462
- WO-A-2004/045618
- WO-A-2005/102349
- CALVO G M ET AL: "IS IT USEFUL TO ADD AN ANTICHOLINERGIC TREATMENT TO BETA2-ADRENERGIC MEDICATION IN ACUTE ASTHMA ATTACK" JOURNAL OF INVESTIGATIONAL ALLERGOLOGY AND CLINICAL IMMUNOLOGY, BARCELONA, ES, Bd. 8, Nr. 1, Januar 1998 (1998-01), Seiten 30-34, XP009018610 ISSN: 1018-9068

## Beschreibung

Die vorliegende Erfindung betrifft eine treibgasfreie Aerosolformulierung, die eine Verbindung der Formel **1,** 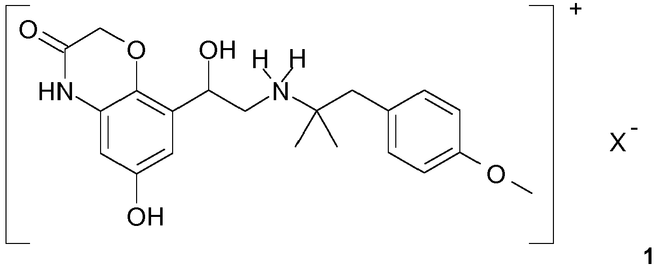 worin X- die in den Ansprüchen und in der Beschreibung genannten Bedeutungen haben kann, und einen weiteren Wirkstoff **2** enthalten, für die Inhalation.

### HINTERGRUND DER ERFINDUNG

EP 0073505 A1 offenbart Benzo-Heterozyklen und deren Verwendung in pharmazeutischen Zubereitungen. WO 01/83462 A beschreibt beta-Mimetika und deren Verwendung als Arzneimittel sowie darüber hinaus deren Kombination mit anderen Substanzen. WO 2004/045618 A offenbart die Nutzung von beta-Agonisten zur Herstellung eines Arzneimittels zur Behandlung von COPD. WO 2005/102349 A beschreibt Arzneimittelkombinationen aus der Kombination von Wirkstoffen ähnlicher Struktur wie in Formel 1 dargestellt und Anticholinergika, jedoch keine spezifische Formulierung enthaltend reines Wasser als Lösungsmittel.

### DETAILLIERTE BESCHREIBUNG DER ERFINDUNG

Die erfindungsgemäßen Arzneimittelformulierungen sind treibgasfreie Arzneimittelformulierungen, enthaltend als Wirkstoff eine Verbindung der Formel **1** 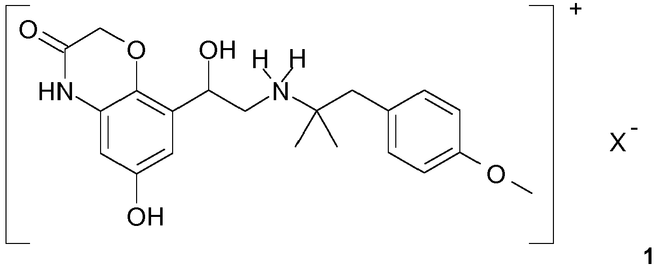 worin
- X-: ein einfach negativ geladenes Anion, bevorzugt ein einfach negativ geladenes Anion ausgewählt aus der Gruppe bestehend aus Chlorid, Bromid, Iodid, Sulfat, Phosphat, Methansulfonat, Nitrat, Maleat, Acetat, Benzoat, Citrat, Salicylat, Trifluoracetat, Fumarat, Tartrat, Oxalat, Succinat, Benzoat und p-Toluolsulfonat,
bedeuten, gegebenenfalls in Form ihrer Tautomere, Enantiomere, Mischungen der Enantiomere, Racemate, Solvate oder Hydrate; einen weiteren Wirkstoff **2.1,** Tiotropiumsalz, , gegebenenfalls in Form seiner Tautomere, Enantiomere, Mischungen der Enantiomere, Racemate, Solvate oder Hydrate; wenigstens eine pharmakologisch verträgliche Säure, gegebenenfalls weitere pharmakologisch verträgliche Hilfsstoffe und/oder Komplexbildner sowie als Lösungsmittel Wasser.

Die Verbindung der Formel 1 kann beschrieben werden als
- 6-Hydroxy-8-{1-hydroxy-2-[2-(4-methoxy-phenyl)-1,1-dimethyl-ethylamino]-ethyl}-4H-benzo[1,4]oxazin-3-on;
jeweils in Form eines Säureadditionssalzes mit einer Säure HX, in der X- eine der vorstehend genannten Bedeutungen haben kann, sowie gegebenenfalls in Form ihrer Tautomere, Enantiomere, Mischungen der Enantiomere, Racemate, Solvate oder Hydrate.

In den erfindungsgemäßen Arzneimittelkombinationen ist der Wirkstoff **2** ein Tiotropiumsalz **(2.1).**

In den vorstehend genannten Salzen von **2.1** stellt das Kation Tiotropium den pharmakologisch aktiven Bestandteil dar. Eine explizite Bezugnahme auf das vorstehend genannte Kation erfolgt durch die Bezeichnungen **2.1'.** Jede Bezugnahme auf die vorstehend genannten Salze **2.1** schließt naturgemäß eine Bezugnahme auf das entsprechende Kation Tiotropium (**2.1')**mit ein.

Unter den Salzen von **2.1** werden erfindungsgemäß diejenigen Verbindungen verstanden, die neben dem Kation Tiotropium (**2.1')**als Gegenion (Anion) Chlorid, Bromid, Iodid, Sulfat, Phosphat, Methansulfonat, Nitrat, Maleat, Acetat, Citrat, Fumarat, Tartrat, Oxalat, Succinat, Benzoat oder p-Toluolsulfonat enthalten, wobei Chlorid, Bromid, Iodid, Sulfat, Methansulfonat oder p-Toluolsulfonat als Gegenionen bevorzugt sind. Von allen Salzen sind die Chloride, Bromide, Iodide und Methansulfonate besonders bevorzugt.

Von besonderer Bedeutung sind Arzneimittelkombinationen, die Tiotropiumsalze (**2.1**), enthalten, wobei die jeweiligen Bromide erfindungsgemäß besonders bedeutsam sind. Von besonderer Bedeutung ist das Tiotropiumbromid (**2.1**).

Die vorstehend genannten Salze können in den erfindungsgemäßen Arzneimittelkombinationen gegebenenfalls in Form ihrer Solvate oder Hydrate, bevorzugt in Form ihrer Hydrate vorliegen. Im Falle des Tiotropiumbromids enthalten die erfindungsgemäßen Arzneimittelkombinationen dieses bevorzugt in Form des kristallinen Tiotropiumbromid Monohydrats, welches aus der WO 02/30928 bekannt ist. Wird das Tiotropiumbromid in den erfindungsgemäßen Arzneimittelkombinationen in wasserfreier Form eingesetzt, so gelangt bevorzugt das wasserfreie kristalline Tiotropiumbromid zur Anwendung, welches aus der WO 03/000265 bekannt ist.

### VERWENDETE BEGRIFFE UND DEFINITIONEN

Unter Säureadditionssalzen mit pharmakologisch unbedenklichen Säuren werden beispielsweise Salze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrobenzoat, Hydrocitrat, Hydrofumarat, Hydrotartrat, Hydrooxalat, Hydrosuccinat, Hydrobenzoat und Hydro-p-toluolsulfonat, bevorzugt Hydrochlorid, Hydrobromid, Hydrosulfat, Hydrophosphat, Hydrofumarat und Hydromethansulfonat verstanden. Von den vorstehend genannten Säureadditionssalzen sind die Salze der Chlorwasserstoffsäure, der Methansulfonsäure, der Benzoesäure und der Essigsäure erfindungsgemäß besonders bevorzugt.

Die erfindungsgemäßen Arzneimittelformulierungen enthalten als Lösungsmittel reines Wasser.

Die Herstellung der erfindungsgemäßen Verbindungen kann in Analogie zu im Stand der Technik bereits bekannten Vorgehensweisen erfolgen. Geeignete Herstellverfahren sind beispielsweise aus der US 4460581 bekannt, auf die an dieser Stelle vollinhaltlich Bezug genommen wird.

Die Verbindungen der Formel **1** können in den erfindungsgemäßen Arzneimittelformulierungen gegebenenfalls in Form ihrer Tautomere enthalten sein. Unter Tautomerie versteht man das Auftreten isomerer Verbindungen, die unter Verschiebung von σ- oder π-Bindungen entstehen und im Gleichgewicht vorliegen können. Beispiele für mögliche tautomere Formen der Verbindungen der Formel **1** sind 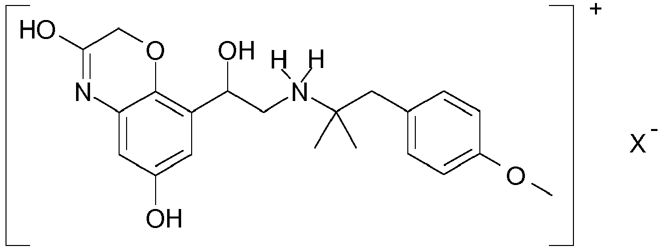 oder auch

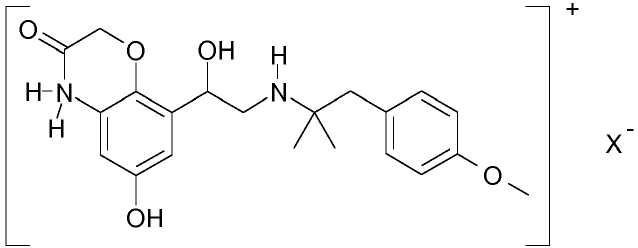

Ein weiterer Aspekt der vorliegenden Erfindung betrifft Arzneimittelformulierungen, die die vorstehend genannten Verbindungen der Formel **1** in Form der einzelnen optischen Isomeren, Mischungen der einzelnen Enantiomeren oder Racemate enthalten. Besonders bevorzugt sind hierbei Arzneimittelformulierungen die die vorstehend genannten Verbindungen der Formel **1** in Form der enantiomerenreinen Verbindungen enthalten, wobei hierbei die R-Enantiomere der Verbindungen der Formel **1** erfindungsgemäß von herausragender Bedeutung ist. Diese R-Enantiomere sind durch die Formel ***R*-1** darstellbar 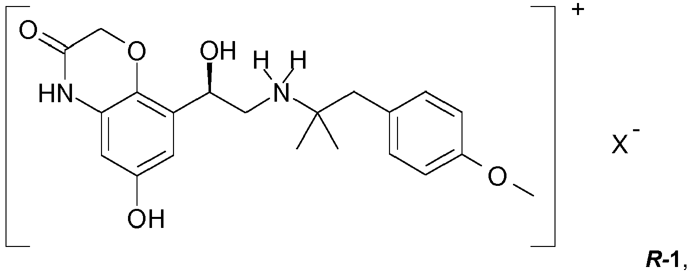 worin X- die vorstehend genannten Bedeutungen haben kann.

Im Rahmen der vorliegenden Erfindung gelangen besonders bevorzugt jene Verbindungen der Formel **1** zur Anwendung, in denen X- ausgewählt ist aus der Gruppe bestehend aus Chlorid, Maleat, Salicylat, Fumarat oder Succinat, gegebenenfalls in Form ihrer Hydrate und Solvate. Erfindungsgemäß besonders bevorzugt im Rahmen der vorliegenden Erfindung sind diejenigen Formulierungen, die die Verbindung der Formel **1,** in der X⁻ für Chlorid steht, enthalten.

Bezugnahmen auf die Verbindung der Formel **1** schließen im Rahmen der vorliegenden Erfindung stets alle möglichen amorphen und kristallinen Modifikationen dieser Verbindung mit ein. Bezugnahmen auf die Verbindung der Formel **1** schließen im Rahmen der vorliegenden Erfindung ferner alle möglichen Solvate und Hydrate, die von dieser Verbindung gebildet werden können mit ein. Eine gegebenenfalls im Rahmen der vorliegenden Erfindung erfolgende Bezugnahme auf die Verbindung **1'** ist als Bezugnahme auf die in den Salzen **1** enthaltene pharmakologisch aktive freie Base der nachstehenden Formel anzusehen 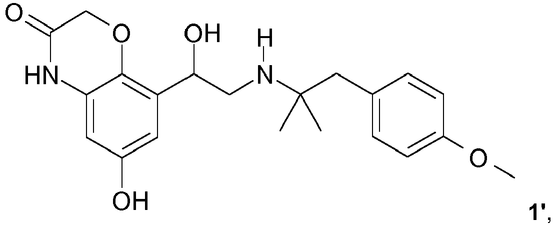 worin X- die vorstehend genannten Bedeutungen haben kann.

Ein weiterer Aspekt der vorliegenden Erfindung betrifft Arzneimittelformulierungen enthaltend einen Wirkstoff **2** und eine freie Base der Formel **1'** worin X- die vorstehend genannten Bedeutungen haben kann, bedeuten, gegebenenfalls in Form ihrer Tautomere, Enantiomere, Mischungen der Enantiomere, Racemate, Solvate oder Hydrate, wenigstens eine pharmakologisch verträgliche Säure, gegebenenfalls weitere pharmakologisch verträgliche Hilfsstoffe und/oder Komplexbildner sowie als Lösungsmittel Wasser.

Ein weiterer Aspekt der vorliegenden Erfindung betrifft die Verwendung der erfindungsgemäßen Arzneimittelformulierungen zur Herstellung eines Arzneimittels zur Behandlung von Atemwegserkrankungen, die ausgewählt sind aus der Gruppe bestehend aus Obstruktive Lungenerkrankungen unterschiedlicher Genese, Lungenemphyseme unterschiedlicher Genese, Restriktive Lungenerkrankungen, Interstitielle Lungenerkrankungen, Zystische Fibrose, Bronchitiden unterschiedlicher Genese, Bronchiektasen, ARDS (adult respiratory distress syndrom) und alle Formen des Lungenödems.

Bevorzugt ist die vorstehend genannte Verwendung zur Herstellung eines Arzneimittels zur Behandlung von Obstruktive Lungenerkrankungen die ausgewählt sind aus der Gruppe bestehend aus Asthma Bronchiale, pädiatrisches Asthma, schweres Asthma, akuter Asthma-Anfall, chronische Bronchitis und chronisch obstruktive Lungenerkrankung (COPD), wobei die Verwendung zur Herstellung eines Arzneimittels zur Behandlung von Asthma Bronchiale oder COPD erfindungsgemäß besonders bevorzugt ist.

Bevorzugt ist ferner die Verwendung der erfindungsgemäßen Arzneimittelformulierungen zur Herstellung eines Arzneimittels zur Behandlung von Lungenemphysemen die ihren Ursprung haben in COPD (chronisch obstruktive pulmonale Erkrankung) oder α1-Proteinase-Inhibitor-Mangel.

Bevorzugt ist ferner die Verwendung der erfindungsgemäßen Arzneimittelformulierungen zur Herstellung eines Arzneimittels zur Behandlung von Restriktiven Lungenerkrankungen, die ausgewählt sind aus der Gruppe bestehend aus Allergische Alveolitis, durch berufliche Noxen ausgelöste restriktive Lungenerkrankungen wie Asbestose oder Silikose und Restriktion aufgrund von Lungentumoren, wie beispielsweise Lymphangiosis carcinomatosa, bronchoalveoläres Karzinom und Lymphome.

Bevorzugt ist ferner die Verwendung der erfindungsgemäßen Arzneimittelformulierungen zur Herstellung eines Arzneimittels zur Behandlung von Interstitiellen Lungenerkrankungen, die ausgewählt sind aus der Gruppe bestehend aus infektiös bedingte Pneumonien, wie beispielsweise aufgrund einer Infektion mit Viren, Bakterien, Pilzen, Protozoen, Helminthen oder anderen Erregern, Pneumonitis aufgrund unterschiedlicher Genese, wie beispielsweise Aspiration und Linksherzinsuffizienz, Strahlen-induzierte Pneumonitis oder Fibrose, Kollagenosen, wie beispielsweise Lupus erythematodes, systemische Sklerodermie oder Sarkoidose, Granulomatosen, wie beispielsweise Morbus Boeck, idiopathische interstitielle Pneumonie oder idiopathische pulmonäre Fibrose (IPF).

Bevorzugt ist ferner die Verwendung der erfindungsgemäßen Arzneimittelformulierungen zur Herstellung eines Arzneimittels zur Behandlung von Zystischer Fibrose bzw. Mukoviszidose.

Bevorzugt ist ferner die Verwendung der erfindungsgemäßen Arzneimittelformulierungen zur Herstellung eines Arzneimittels zur Behandlung von Bronchitiden, wie beispielsweise Bronchitis aufgrund bakterieller oder viraler Infektion, Allergische Bronchitis und Toxische Bronchitis.

Bevorzugt ist ferner die Verwendung der erfindungsgemäßen Arzneimittelformulierungen zur Herstellung eines Arzneimittels zur Behandlung von Bronchiektasen.

Bevorzugt ist ferner die Verwendung der erfindungsgemäßen Arzneimittelformulierungen zur Herstellung eines Arzneimittels zur Behandlung von ARDS (adult respiratory distress syndrom).

Bevorzugt ist ferner die Verwendung der erfindungsgemäßen Arzneimittelformulierungen zur Herstellung eines Arzneimittels zur Behandlung von Lungenödemen, beispielsweise toxischer Lungenödeme nach Aspiration oder Inhalation von toxischen Substanzen und Fremdstoffen.

Besonders bevorzugt betrifft die vorliegende Erfindung die Verwendung der erfindungsgemäßen Arzneimittelformulierungen zur Herstellung eines Arzneimittels zur Behandlung von Asthma oder COPD. Von besonderer Bedeutung ist ferner die vorstehend genannte Verwendung zur Herstellung eines Arzneimittels zur einmal täglichen Behandlung von entzündlichen und obstruktiven Atemwegserkrankungen, besonders bevorzugt zur einmal täglichen Behandlung von Asthma oder COPD.

Ferner betrifft die vorliegende Erfindung ein Verfahren zur Behandlung der vorstehend genannten Erkrankungen, dadurch gekennzeichnet, dass eine oder mehrere der vorstehend genannten erfindungsgemäßen Arzneimittelformulierungen in therapeutisch wirksamen Mengen appliziert werden.

Die vorliegende Erfindung beschäftigt sich mit inhalativ applizierbaren flüssigen Wirkstoffformulierungen dieser Verbindungen, wobei die erfindungsgemäßen flüssigen Formulierungen hohen Qualitätsstandards genügen müssen. Die erfindungsgemäßen Formulierungen können dabei peroral oder pernasal inhaliert werden. Um eine optimale Wirkstoffverteilung der Wirksubstanzen in der Lunge zu erhalten, bietet sich die Applikation einer flüssigen, auf Treibgase verzichtenden, Formulierung mittels dafür geeigneter Inhalatoren an. Die inhalative Applikation einer solchen Formulierung kann sowohl auf oralem als auch auf nasalem Weg erfolgen. Besonders geeignet sind solche Inhalatoren, die eine kleine Menge einer flüssigen Formulierung in der therapeutisch notwendigen Dosierung binnen weniger Sekunden in ein therapeutisch-inhalativ geeignetes Aerosol vernebeln können. Im Rahmen der vorliegenden Erfindung sind solche Vernebler bevorzugt, bei denen bereits eine Menge von weniger als 100 Mikrolitern, bevorzugt weniger als 50 Mikrolitern, ganz bevorzugt weniger als 25 Mikrolitern Wirkstofflösung mit bevorzugt einem Hub oder zwei Hüben zu einem Aerosol mit einer durchschnittlichen Teilchengröße (bzw. Teilchendurchmesser) von weniger als 20 Mikrometern, bevorzugt weniger als 10 Mikrometern, so vernebelt werden können, dass der inhalierbare Anteil des Aerosols bereits der therapeutisch wirksamen Menge entspricht.

Eine derartige Vorrichtung zur treibgasfreien Verabreichung einer dosierten Menge eines flüssigen Arzneimittels zur inhalativen Anwendung, wird beispielsweise in der internationalen Patentanmeldung WO 91/14468 "Atomizing Device and Methods" als auch in der WO 97/12687, dort Figuren 6a und 6b und der dazugehörigen Beschreibung, ausführlich beschrieben. In einem solchen Vernebler wird eine Arzneimittellösung mittels hohen Drucks von bis zu 500 bar in ein lungengängiges Aerosol überführt und versprüht. Auf die genannten Referenzen wird im Rahmen der vorliegenden Erfindungsbeschreibung ausdrücklich in Gänze Bezug genommen.

In solchen Inhalatoren werden die Lösungsformulierungen in einem Reservoir gelagert. Dabei ist es notwendig, dass die verwendeten Wirkstoffformulierungen eine ausreichende Lagerstabilität aufweisen und gleichzeitig so beschaffen sind, dass sie dem medizinischen Zweck entsprechend möglichst ohne weitere Manipulation, direkt appliziert werden können. Ferner dürfen sie keine Bestandteile aufweisen, die so mit dem Inhalator wechselwirken können, dass der Inhalator oder die pharmazeutische Qualität der Lösung, respektive des erzeugten Aerosols, Schaden nehmen könnte.

Zur Vernebelung der Lösung wird eine spezielle Düse verwendet, wie sie beispielsweise die WO 94/07607 oder die WO 99/16530 beschreibt. Auf beide wird hiermit ausdrücklich Bezug genommen.

Es ist Aufgabe der vorliegenden Erfindung, eine wässrige Formulierung der Verbindung der Formel **1** gegebenenfalls in Form ihrer Tautomere, Enantiomere, Mischungen der Enantiomere, Racemate, Solvate oder Hydrate; einen weiteren Wirkstoff **2,** ein Tiotropiumsalz, gegebenenfalls in Form seiner Solvate oder Hydrate, bereitzustellen, welche den hohen Standards genügt, die notwendig sind, um eine Lösung mittels der eingangs genannten Inhalatoren optimal vernebeln zu können. Die erfindungsgemäßen Wirkstoffformulierungen müssen dabei auch eine ausreichende pharmazeutische Qualität aufweisen, d.h. sie sollten über eine Lagerzeit von einigen Jahren, bevorzugt von mindestens einem Jahr, stärker bevorzugt von zwei Jahren pharmazeutisch stabil sein.

Diese treibgasfreien Lösungsformulierungen müssen ferner mittels eines Inhalators unter Druck vernebelt werden können, wobei die im generierten Aerosol ausgebrachte Masse reproduzierbar innerhalb eines definierten Bereichs liegt.

Erfindungsgemäß enthält die Formulierung bevorzugt den Wirkstoff **2** und die Verbindung der Formel **1.** Allerdings kann die Formulierung auch ein Gemisch verschiedener Salze der Formel **1** enthalten. Enthalten die erfindungsgemäßen Arzneimittelformulierungen verschiedene Salze der Formel **1** sind erfindungsgemäß diejenigen Formulierungen bevorzugt, in denen die verschiedenen Salze unterschiedliche Salze derselben freien Base der Formel **1'** darstellen.

Die Konzentration der Verbindung der Formel **1** bezogen auf den Anteil an pharmakologisch wirksamer freien Base **1'** in der erfindungsgemäßen Arzneimittelformulierung liegt erfindungsgemäß bei etwa 0,1 bis 2000 mg pro 100 ml, bevorzugt bei etwa 0,5 bis 1100 mg pro 100 ml, besonders bevorzugt 0,75 bis 500 mg pro 100 ml. Besonders bevorzugt enthalten 100ml der erfindungsgemäßen Formulierungen etwa 1 bis etwa 250 mg **1'.**

Die Konzentration der Verbindung der Formel **2** bezogen auf den Anteil an pharmakologisch wirksamem freien Kation des Salzes **2.1** in der erfindungsgemäßen Arzneimittelformulierung liegt erfindungsgemäß bei etwa 0,1 bis 2000 mg pro 100 ml, bevorzugt bei etwa 0,5 bis 1100 mg pro 100 ml, besonders bevorzugt 0,75 bis 500 mg pro 100 ml. Besonders bevorzugt enthalten 100 ml der erfindungsgemäßen Formulierungen etwa 1 bis etwa 250 mg des freien Kations des Salzes **2.1.**

Der pH-Wert der erfindungsgemäßen Formulierung liegt erfindungsgemäß bevorzugt in einem Bereich von 2,0 und 6,5 bevorzugt zwischen 2,5 und 3,5, besonders bevorzugt zwischen etwa 2,7 und 3,1 in rein wässrigen Lösungen.

Der pH-Wert wird durch Zugabe von pharmakologisch verträglichen Säuren eingestellt. Hierzu können pharmakologisch verträgliche anorganische oder organische Säuren zur Anwendung gelangen. Beispiele für bevorzugte anorganische Säuren sind ausgewählt aus der Gruppe bestehend aus Salzsäure, Bromwasserstoffsäure, Salpetersäure, Schwefelsäure und Phosphorsäure. Beispiele für besonders geeignete organische Säuren sind ausgewählt aus der Gruppe bestehend aus Ascorbinsäure, Zitronensäure, Äpfelsäure, Weinsäure, Maleinsäure, Bernsteinsäure, Fumarsäure, Essigsäure, Ameisensäure, Propionsäure, Sorbinsäure, Benzoesäure, Methansulfonsäure and Benzolsulfonsäure . Bevorzugte anorganische Säuren sind Salzsäure, Phosphorsäure und Schwefelsäure, wobei der Salzsäure erfindungsgemäß besondere Bedeutung zukommt. Unter den organischen Säuren sind Ascorbinsäure, Fumarsäure, Methansulfonsäure und Zitronensäure bevorzugt, wobei Zitronensäure erfindungsgemäß besonders bevorzugt ist. Gegebenenfalls können auch Gemische der genannten Säuren eingesetzt werden, insbesondere in Fällen von Säuren, die neben ihren Säuerungseigenschaften auch andere Eigenschaften, z.B. als Geschmackstoffe oder Antioxidantien besitzen, wie beispielsweise Zitronensäure oder Ascorbinsäure. Gegebenenfalls können auch pharmakologisch verträgliche Basen zum genauen Austitrieren des pH-Wertes eingesetzt werden. Als Basen eignen sich beispielsweise Alkalihydroxide und Alkalicarbonate. Bevorzugtes Alkaliion ist Natrium. Werden solche Basen verwendet, ist darauf zu achten, dass auch die daraus resultierenden Salze, die dann in der fertigen Arzneimittelformulierung enthalten sind, mit der oben genannten Säure pharmakologisch akzeptabel sind.

Die erfindungsgemäßen Formulierungen können als weitere Bestandteile Komplexbildner enthalten. Unter Komplexbildner werden im Rahmen der vorliegenden Erfindung Moleküle verstanden, die in der Lage sind Komplexbindungen einzugehen. Bevorzugt sollen durch diese Verbindungen Kationen, besonders bevorzugt metallische Kationen komplexiert werden. Die erfindungsgemäßen Formulierungen enthalten als Komplexbildner bevorzugt Editinsäure (EDTA) oder ein bekanntes Salz davon, z.B. Natrium-EDTA, bzw. Dinatrium-EDTA. Bevorzugt wird Dinatriumedetat, gegebenenfalls in Form seiner Hydrate, besonders bevorzugt in Form seines Dihydrats eingesetzt. Werden im Rahmen der erfindungsgemäßen Formulierungen Komplexbildner verwendet, so liegt deren Gehalt bevorzugt in einem Bereich von 0,1 bis 50 mg pro 100 ml, besonders bevorzugt in einem Bereich von 0,5 bis 25 mg pro 100 ml der erfindungsgemäßen Formulierung. Vorzugsweise enthalten die erfindungsgemäßen Formulierungen einen Komplexbildner in einer Menge von etwa 0,75 bis 15 mg pro 100 ml, besonders bevorzugt von etwa 1 bis 12 mg pro 100 ml.

Analoges wie bereits für Dinatriumedetat ausgeführt, gilt auch für mögliche, mit EDTA oder seinen Salzen vergleichbare Zusatzstoffe, die komplexbildende Eigenschaften aufweisen und anstelle dessen verwendet werden können, wie beispielsweise Nitrilotriessigsäure und deren Salze.

Der erfindungsgemäßen Formulierung können weitere pharmakologisch verträgliche Hilfsstoffe zugesetzt werden. Unter Hilfs- und Zusatzstoffen wird in diesem Zusammenhang jeder pharmakologisch verträgliche und therapeutisch sinnvolle Stoff verstanden, der kein Wirkstoff ist, aber zusammen mit dem Wirkstoff in dem pharmakologisch geeigneten Lösungsmittel formuliert werden kann, um die qualitativen Eigenschaften der Wirkstoffformulierung zu verbessern. Bevorzugt entfalten diese Stoffe keine oder im Kontext mit der angestrebten Therapie keine nennenswerte oder zumindest keine unerwünschte pharmakologische Wirkung. Zu den Hilfs- und Zusatzstoffen zählen z.B. Stabilisatoren, Antioxidantien und/oder Konservierungsstoffe, die die Verwendungsdauer der fertigen Arzneimittelformulierung verlängern sowie Geschmackstoffe Vitamine und/oder sonstige dem Stand der Technik bekannte Zusatzstoffe. Zu den Zusatzstoffen zählen auch pharmakologisch unbedenkliche Salze wie beispielsweise Natriumchlorid.

Zu den bevorzugten Hilfsstoffen zählen Antioxidantien, wie beispielsweise Ascorbinsäure, sofern nicht bereits für die Einstellung des pH-Werts verwendet, Propylgallat, BHA (Butylhydroxyanisol), BHT (Butylhydroxytoluol), TBHQ (tert-Butylhydroxyquinon), Vitamin A, Vitamin E, Tocopherole und ähnliche im menschlichen Organismus vorkommende Vitamine oder Provitamine.

Konservierungsstoffe können eingesetzt werden, um die Formulierung vor Kontamination mit pathogenen Keimen zu schützen. Als Konservierungsstoffe eignen sich die dem Stand der Technik bekannten, insbesondere Benzalkoniumchlorid oder Benzoesäure bzw. Benzoate wie Natriumbenzoat in den aus dem Stand der Technik bekannten Konzentrationen. Bevorzugt wird der erfindungsgemäßen Formulierung Benzalkoniumchlorid beigemischt. Die Menge des Benzalkoniumchlorids beträgt dabei zwischen 1 mg und 50 mg pro 100 ml Formulierung, bevorzugt etwa 2 bis 15 mg pro 100 ml, besonders bevorzugt etwa 3 bis 12 mg pro 100 ml, besonders bevorzugt etwa 4 bis 11 mg pro 100 ml der erfindungsgemäßen Formulierung. Benzalkoniumchlorid kann erfindungsgemäß auch im Gemisch mit anderen Konservierungsstoffen zum Einsatz gelangen.

Bevorzugte Formulierungen enthalten außer dem Lösungsmittel Wasser, den Verbindungen der Formel **1** und Wirkstoff **2** nur noch Benzalkoniumchlorid, Natriumedetat und die zum Einstellen des pH-Werts notwendige Säure.

Die erfindungsgemäßen Arzneimittelformulierungen werden bevorzugt in einem Inhalator der vorstehend beschriebenen Art verwendet, um daraus die erfindungsgemäßen treibgasfreien Aerosole herzustellen. An dieser Stelle sei deshalb noch einmal ausdrücklich auf die eingangs beschriebenen Patentdokumente verwiesen, auf die hiermit vollinhaltlich Bezug genommen wird. Wie eingangs geschildert wird eine weiterentwickelte Ausführungsform des bevorzugten Inhalators in der WO 97/12687 (siehe dort insbesondere Figuren 6a und 6b und die diesbezüglichen Beschreibungsteile) offenbart. Dieser Vernebler (Respimat®) kann vorteilhaft zur Erzeugung der erfindungsgemäßen inhalierbaren Aerosole eingesetzt werden. Aufgrund seiner zylinderähnlichen Form und einer handlichen Größe von weniger als 9 bis 15 cm in der Länge und 2 bis 4 cm in der Breite kann dieses Device jederzeit vom Patienten mitgeführt werden. Der Vernebler versprüht ein definiertes Volumen der Arzneimittelformulierung unter Anwendung hoher Drücke durch kleine Düsen, so dass inhalierbare Aerosole entstehen. Im wesentlichen besteht der bevorzugte Zerstäuber aus einem Gehäuseoberteil, einem Pumpengehäuse, einer Düse, einem Sperrspannwerk, einem Federgehäuse, einer Feder und einem Vorratsbehälter, gekennzeichnet durch
- ein Pumpengehäuse, das im Gehäuseoberteil befestigt ist, und das an seinem
- einen Ende einen Düsenkörper mit der Düse bzw. Düsenanordnung trägt,
- einen Hohlkolben mit Ventilkörper,
- einen Abtriebsflansch, in dem der Hohlkolben befestigt ist, und der sich im Gehäuseoberteil befindet,
- ein Sperrspannwerk, das sich im Gehäuseoberteil befindet,
- ein Federgehäuse mit der darin befindlichen Feder, das am Gehäuseoberteil mittels eines Drehlagers drehbar gelagert ist,
- ein Gehäuseunterteil, das auf das Federgehäuse in axialer Richtung aufgesteckt ist.

Der Hohlkolben mit Ventilkörper entspricht einer in der WO 97/12687 offenbarten Vorrichtungen. Er ragt teilweise in den Zylinder des Pumpengehäuses hinein und ist im Zylinder axial verschiebbar angeordnet. Insbesondere wird auf die Figuren 1-4 - insbesondere Figur 3 - und die dazugehörigen Beschreibungsteile der o.g. Internationalen Patentanmeldung Bezug genommen. Der Hohlkolben mit Ventilkörper übt auf seiner Hochdruckseite zum Zeitpunkt des Auslösens der Feder einen Druck von 5 bis 60 MPa (etwa 50 bis 600 bar), bevorzugt 10 bis 60 MPa (etwa 100 bis 600 bar) auf das Fluid, die abgemessene Wirkstofflösung aus. Dabei werden Volumina von 10 bis 50 Mikroliter bevorzugt, besonders bevorzugt sind Volumina von 10 bis 20 Mikroliter, ganz besonders bevorzugt ist ein Volumen von 10 bis 15 Mikroliter pro Hub.

Der Ventilkörper ist bevorzugt an dem Ende des Hohlkolbens angebracht, das dem Düsenkörper zugewandt ist.

Die Düse im Düsenkörper ist bevorzugt mikrostrukturiert, d.h. durch Mikrotechnik hergestellt. Mikrostrukturierte Düsenkörper sind beispielsweise in der WO-99/16530 offenbart; auf diese Schrift wird hiermit inhaltlich Bezug genommen, insbesondere auf die dort offenbarte Figur 1 und deren Beschreibung. Der Düsenkörper besteht z.B. aus zwei fest miteinander verbundenen Platten aus Glas und/oder Silizium, von denen wenigstens eine Platte einen oder mehrere mikrostrukturierte Kanäle aufweist, die die Düseneinlassseite mit der Düsenauslassseite verbinden. Auf der Düsenauslassseite ist mindestens eine runde oder nicht-runde Öffnung von 2 bis 10 Mikrometer Tiefe und 5 bis 15 Mikrometern Breite, wobei die Tiefe bevorzugt bei 4, 5 bis 6,5 Mikrometern und die Länge bei 7 bis 9 Mikrometern beträgt. Im Fall von mehreren Düsenöffnungen, bevorzugt sind zwei, können die Strahlrichtungen der Düsen im Düsenkörper parallel zueinander verlaufen oder sie sind in Richtung Düsenöffnung gegeneinander geneigt. Bei einem Düsenkörper mit mindestens zwei Düsenöffnungen auf der Auslassseite können die Strahlrichtungen mit einem Winkel von 20 Grad bis 160 Grad gegeneinander geneigt sein, bevorzugt wird ein Winkel von 60 bis 150 Grad, insbesondere bevorzugt 80 bis 100°. Die Düsenöffnungen sind bevorzugt in einer Entfernung von 10 bis 200 Mikrometern angeordnet, stärker bevorzugt in einer Entfernung von 10 bis 100 Mikrometer, besonders bevorzugt 30 bis 70 Mikrometer. Am stärksten bevorzugt sind 50 Mikrometer.

Die Strahlrichtungen treffen sich dementsprechend in der Umgebung der Düsenöffnungen.

Die flüssige Arzneimittelformulierung trifft wie bereits erwähnt mit einem Eingangsdruck von bis zu 600 bar, bevorzugt 200 bis 300 bar auf den Düsenkörper und wird über die Düsenöffnungen in ein inhalierbares Aerosol zerstäubt. Die bevorzugten Teilchengrößen des Aerosols liegen bei bis zu 20 Mikrometern, bevorzugt 3 bis 10 Mikrometern.

Das Sperrspannwerk enthält eine Feder, bevorzugt eine zylindrische schraubenförmige Druckfeder, als Speicher für die mechanische Energie. Die Feder wirkt auf den Abtriebsflansch als Sprungstück, dessen Bewegung durch die Position eines Sperrglieds bestimmt wird. Der Weg des Abtriebsflansches wird durch einen oberen und einen unteren Anschlag präzise begrenzt. Die Feder wird bevorzugt über ein kraftübersetzendes Getriebe, z.B. ein Schraubschubgetriebe, durch ein äußeres Drehmoment gespannt, das beim Drehen des Gehäuseoberteils gegen das Federgehäuse im Gehäuseunterteil erzeugt wird. In diesem Fall enthalten das Gehäuseoberteil und der Abtriebsflansch ein ein- oder mehrgängiges Keilgetriebe.

Das Sperrglied mit einrückenden Sperrflächen ist ringförmig um den Abtriebsflansch angeordnet. Es besteht z.B. aus einem in sich radial elastisch verformbaren Ring aus Kunststoff oder aus Metall. Der Ring ist in einer Ebene senkrecht zur Zerstäuberachse angeordnet. Nach dem Spannen der Feder schieben sich die Sperrflächen des Sperrgliedes in den Weg des Abtriebsflansches und verhindern das Entspannen der Feder. Das Sperrglied wird mittels einer Taste ausgelöst. Die Auslösetaste ist mit dem Sperrglied verbunden oder gekoppelt. Zum Auslösen des Sperrspannwerkes wird die Auslösetaste parallel zur Ringebene, und zwar bevorzugt in den Zerstäuber hinein, verschoben; dabei wird der verformbare Ring in der Ringebene verformt. Konstruktive Details des Sperrspannwerkes sind in der WO 97/20590 beschrieben.

Das Gehäuseunterteil wird in axialer Richtung über das Federgehäuse geschoben und verdeckt die Lagerung, den Antrieb der Spindel und den Vorratsbehälter für das Fluid.

Beim Betätigen des Zerstäubers wird das Gehäuseoberteil gegen das Gehäuseunterteil gedreht, wobei das Gehäuseunterteil das Federgehäuse mitnimmt. Dabei wird die Feder über das Schraubschubgetriebe zusammengedrückt und gespannt, und das Sperrwerk rastet selbsttätig ein. Der Drehwinkel ist bevorzugt ein ganzzahliger Bruchteil von 360 Grad, z.B. 180 Grad. Gleichzeitig mit dem Spannen der Feder wird das Abtriebsteil im Gehäuseoberteil um einen vorgegebenen Weg verschoben, der Hohlkolben wird innerhalb des Zylinders im Pumpengehäuse zurückgezogen, wodurch eine Teilmenge des Fluids aus dem Vorratsbehälter in den Hochdruckraum vor der Düse eingesaugt wird.

In den Zerstäuber können gegebenenfalls nacheinander mehrere das zu zerstäubende Fluid enthaltende austauschbare Vorratsbehälter eingeschoben und benutzt werden. Der Vorratsbehälter enthält die erfindungsgemäße Aerosolzubereitung.

Der Zerstäubungsvorgang wird durch leichtes Eindrücken der Auslösetaste eingeleitet. Dabei gibt das Sperrwerk den Weg für das Abtriebsteil frei. Die gespannte Feder schiebt den Kolben in den Zylinder des Pumpengehäuses hinein. Das Fluid tritt aus der Düse des Zerstäubers in zerstäubter Form aus.

Weitere konstruktive Details sind in den PCT-Anmeldungen WO 97/12683 und WO 97/20590 offenbart, auf die hiermit inhaltlich Bezug genommen wird.

Die Bauteile des Zerstäubers (Vernebiers) sind aus einem der Funktion entsprechend geeignetem Material. Das Gehäuse des Zerstäubers und - so weit es die Funktion erlaubt - auch andere Teile sind bevorzugt aus Kunststoff, z.B. im Spritzgießverfahren, hergestellt. Für medizinische Zwecke werden physiologisch unbedenkliche Materialien verwendet.

In den Figuren 6 a/b der WO 97/12687, ist der Vernebler (Respimat®) beschrieben, mit dem die erfindungsgemäßen wässrigen Aerosolzubereitungen vorteilhaft inhaliert werden können. Figur 6 a zeigt einen Längsschnitt durch den Zerstäuber bei gespannter Feder, Figur 6 b zeigt einen Längsschnitt durch den Zerstäuber bei entspannter Feder.

Das Gehäuseoberteil (51) enthält das Pumpengehäuse (52), an dessen Ende der Halter (53) für die Zerstäuberdüse angebracht ist. In dem Halter befindet sich der Düsenkörper (54) und ein Filter (55). Der im Abtriebsflansch (56) des Sperrspannwerkes befestigte Hohlkolben (57) ragt teilweise in den Zylinder des Pumpengehäuses hinein. An seinem Ende trägt der Hohlkolben den Ventilkörper (58). Der Hohlkolben ist mittels der Dichtung (59) abgedichtet. Innerhalb des Gehäuseoberteils befindet sich der Anschlag (60), an dem der Abtriebsflansch bei entspannter Feder anliegt. Am Abtriebsflansch befindet sich der Anschlag (61), an dem der Abtriebsflansch bei gespannter Feder anliegt. Nach dem Spannen der Feder schiebt sich das Sperrglied (62) zwischen den Anschlag (61) und eine Abstützung (63) im Gehäuseoberteil. Die Auslösetaste (64) steht mit dem Sperrglied in Verbindung. Das Gehäuseoberteil endet im Mundstück (65) und ist mit der aufsteckbaren Schutzkappe (66) verschlossen.

Das Federgehäuse (67) mit Druckfeder (68) ist mittels der Schnappnasen (69) und Drehlager am Gehäuseoberteil drehbar gelagert. Über das Federgehäuse ist das Gehäuseunterteil (70) geschoben. Innerhalb des Federgehäuses befindet sich der austauschbare Vorratsbehälter (71) für das zu zerstäubende Fluid (72). Der Vorratsbehälter ist mit dem Stopfen (73) verschlossen, durch den der Hohlkolben in den Vorratsbehälter hineinragt und mit seinem Ende in das Fluid (Vorrat an Wirkstofflösung) eintaucht.

In der Mantelfläche des Federgehäuses ist die Spindel (74) für das mechanische Zählwerk angebracht. An dem Ende der Spindel, das dem Gehäuseoberteil zugewandt ist, befindet das Antriebsritzel (75). Auf der Spindel sitzt der Reiter (76).

Der oben beschriebene Vernebler ist geeignet, die erfindungsgemäßen Aerosolzubereitungen zu einem für die Inhalation geeignetem Aerosol zu vernebeln.

Wird die erfindungsgemäße Formulierung mittels der vorstehend beschriebenen Technik (Respimat®) vernebelt, sollte die ausgebrachte Masse bei wenigstens 97%, bevorzugt wenigstens 98% aller Betätigungen des Inhalators (Hub oder Hübe) einer definierten Menge mit einem Toleranzbereichs von maximal 25%, bevorzugt 20% dieser Menge entsprechen. Bevorzugt werden pro Hub zwischen 5 und 30 mg Formulierung als definierte Masse ausgebracht, besonders bevorzugt zwischen 5 und 20 mg.

Die erfindungsgemäße Formulierung kann auch mittels anderer als der vorstehend beschriebenen Inhalatoren, beispielsweise Jet-Stream-Inhalatoren, vernebelt werden.

Die vorliegende Erfindung betrifft ferner ein Inhalationskit bestehend aus einer der vorstehend beschriebenen erfindungsgemäßen Arzneimittelzubereitungen und einem zur Vernebelung dieser Arzneimittelformulierung geeigneten Inhalator. Die vorliegende Erfindung betrifft bevorzugt ein Inhalationskit bestehend aus einer der vorstehend beschriebenen erfindungsgemäßen Arzneimittelzubereitungen und dem vorstehend beschriebenen Inhalator Respimat®.

Soll die Formulierung mittels des oben beschriebenen Respimat®-Geräts nasal appliziert werden, so kann dieser Zerstäuber mit einem Aufsatz auf dem Mundstück versehen werden, welcher ähnlich einer zylindrischen Pyramide, d.h. einer Pyramide mit rundem oder ovalen Querschnitt bzw. ein sich verjüngender, runden oder ovalen Zylinder, aufgebaut ist. Dieser Aufsatz ist dann innen hohl und weist zwei Öffnungen auf. Die eine Öffnungen kann auf das Mundstück aufgesetzt werden und die andere Öffnung am spitzen Ende kann in ein Nasenloch eingeführt werden.
Damit weist dieser Aufsatz bevorzugt die Form einer Tülle herkömmlicher Nasensprays auf. Der Aufsatz kann so ausgebildet sein, dass er lösbar mit dem Mundstück oder unlösbar mit diesem verbunden ist. Ein solcher Aufsatz kann auch das Mundstück ersetzen.

Die nachstehend ausgeführten Formulierungsbeispiele dienen der weitergehenden Erläuterung ohne den Gegenstand der vorliegenden Erfindung auf die exemplarisch dargestellten Zusammensetzungen zu beschränken.

### BEISPIELE

Wie bereits erwähnt können die Verbindungen der Formel **1** in an sich bekannter Weise hergestellt werden. Beispielhaft genannte und im Rahmen der Erfindung bevorzugte Verbindungen sind im Folgenden aufgelistet. Bevorzugt sind somit Arzneimittelformulierungen, die einen Wirkstoff **2** und die Verbindung der Formel **1** enthalten, die folgendermaßen bezeichnet werden kann: 6-Hydroxy-8-{1-hydroxy-2-[2-(4-methoxyphenyl)-1,1-dimethyl-ethylamino]-ethyl}-4H-benzo[1,4]oxazin-3-on-Hydrochlorid **(Beispiel 3)**
gegebenenfalls in Form eines Säureadditionssalzes mit einer Säure HX, in der X- eine der vorstehend genannten Bedeutungen haben kann, sowie gegebenenfalls in Form ihrer Tautomere, Enantiomere, Mischungen der Enantiomere, Racemate, Solvate oder Hydrate.

In der nachstehenden Tabelle sind erfindungsgemäße Formulierungsbeispiele zusammengefasst. Dabei bedeutet die Abkürzung BA-CI Benzalkoniumchlorid und EDTA ist gleichzusetzen mit Dinatriumedetat-Dihydrat.

Die genannten Wirkstoffe **1** und **2.1** werden dabei in Form von Salzen und oder Hydraten eingesetzt, sind hier aber bezogen auf die Masse der freien Base von **1** und des freien Kations von **2.1** angegeben. Die Verbindung **1** wird in den unten gezeigten Beispielen als Hydrochlorid, Hydrotetraflouroacetat oder Hydromethansulfonat eingesetzt, die Verbindung **2** als ein Monohydrat des Bromids.

In der nachstehenden Tabelle sind erfindungsgemäße Formulierungsbeispiele des R-Enantiomers der Verbindung Beispiel 3 und dem Wirkstoff **2.1** in Form von Base und Kation zusammengefasst. 100 ml Arzneimittelformulierung enthalten in gereinigtem Wasser bzw. Wasser für Injektionszwecke:

| **Beispiel** | **1'(Base) (mg)** | **2.1'(Kation) (mg)** | **BA-CI (mg)** | **EDTA (mg)** | **pH Wert (HCl)** |
|---|---|---|---|---|---|
| 1. | 1000 | 1000 | - | 2 | 2,9 |
| 2. | 500 | 500 | 10 | 10 | 2,7 |
| 3. | 500 | 250 | 4 | 10 | 2,9 |
| 4. | 500 | 250 | 5 | 10 | 2,9 |
| 5. | 500 | 85 | 10 | 11 | 2,9 |
| 6. | 250 | 500 | 10 | 9 | 3,1 |
| 7. | 85 | 500 | 8 | 10 | 2,9 |
| 8. | 200 | 45 | 15 | - | 2,9 |
| 9. | 200 | 23 | 11 | 12 | 2,7 |
| 10. | 200 | 23 | 10 | 10 | 2,7 |
| 11. | 90 | 23 | 10 | 10 | 2,8 |
| 12. | 90 | 23 | 10 | 5 | 3,1 |
| 13. | 45 | 23 | 10 | 10 | 2,9 |
| 14. | 45 | 23 | 10 | 5 | 3,0 |
| 15. | 23 | 23 | 10 | 10 | 2,9 |
| 16. | 23 | 23 | 10 | 5 | 2,9 |
| 17. | 23 | 45 | 11 | 25 | 3,0 |
| 18. | 23 | 45 | 10 | 10 | 3,0 |
| 19. | 9 | 23 | 10 | 10 | 3,1 |
| 20. | 11 | 23 | 10 | 25 | 3,1 |
| 21. | 9 | 23 | 10 | 5 | 2,8 |
| 22. | 5 | 23 | 10 | 10 | 3,1 |
| 23. | 5 | 23 | 10 | 50 | 3,1 |
| 24. | 5 | 23 | 10 | 5 | 2,7 |
| 25. | 5 | 45 | 12 | 5 | 3,0 |
| 26. | 1 | 1 | 10 | 7 | 3,5 |
| 27. | 1 | 1 | 10 | 7 | 3,0 |
| 28. | 0,1 | 0,1 | 10 | 15 | 3,5 |
| 29. | 0,1 | 0,1 | 10 | 15 | 3,0 |

## Patentansprüche

1. Arzneimittelformulierung, enthaltend als Wirkstoff eine Verbindung der Formel **1** 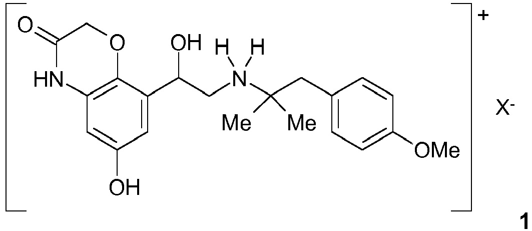 worin
X- bedeutet ein einfach negativ geladenes Anion, bevorzugt ein einfach negativ geladenes Anion ausgewählt aus der Gruppe bestehend aus Chlorid, Bromid, Iodid, Sulfat, Phosphat, Methansulfonat, Nitrat, Maleat, Acetat, Benzoat, Citrat, Salicylat, Trifluoracetat, Fumarat, Tartrat, Oxalat, Succinat, Benzoat und p-Toluolsulfonat;
worin die erfindungsgemäßen Arzneimittelformulierung enthält
• 1 bis 250 mg freien Base **1'** pro 100 ml Lösung,
• 1 bis 250 mg des freien Kations des Tiotropiumsalzes **2.1'** pro 100 ml Lösung,
• reines Wasser als Lösungsmittel,
• mindestens eine pharmakologisch verträgliche Säure,
• wahlweise weitere pharmakologische verträgliche Hilfsstoffe und/oder Komplexbildner.
worin die Arzneimittelformulierung ein pH von 2,5 bis 3,5 hat.

2. Arzneimittelformulierung nach Anspruch 1, worin die pharmakologisch verträgliche Säure ausgewählt ist aus den anorganischen Säuren Salzsäure, Bromwasserstoffsäure, Salpetersäure, Schwefelsäure und Phosphorsäure oder aus den organischen Säuren Ascorbinsäure, Zitronensäure, Äpfelsäure, Weinsäure, Maleinsäure, Bernsteinsäure, Fumarsäure, Essigsäure, Ameisensäure, Propionsäure, Sorbinsäure, Benzoesäure, Methansulfonsäure und Benzolsulfonsäure.

3. Arzneimittelformulierung nach einem der Ansprüche 1 bis 2, **dadurch gekennzeichnet, dass** sie als Hilfsstoff Benzalkoniumchlorid enthalten.

4. Arzneimittelformulierung nach Anspruch 3, **dadurch gekennzeichnet dass** der Gehalt an Benzalkoniumchlorid 1 bis 50 mg pro 100 ml Lösung beträgt.

5. Arzneimittelformulierung nach einem der Ansprüche 1 bis 4, **dadurch gekennzeichnet, dass** sie als weiteren pharmakologisch verträglichen Hilfsstoff einen Antioxidant enthält.

6. Arzneimittelformulierung nach einem der Ansprüche 1 bis 5, **dadurch gekennzeichnet, dass** sie als weiteren pharmakologisch verträglichen Hilfsstoff einen Antioxidant ausgewählt aus der Gruppe bestehen aus Ascorbinsäure, Propylgallat, Butylhydroxyanisol, Butylhydroxytoluol, tert-Butylhydroxyquinon und Tocopherole enthält.

7. Arzneimittelformulierung nach einem der Ansprüche 1 bis 6, **dadurch gekennzeichnet, dass** sie als weiteren Bestandteil einen Komplexbildner enthalten.

8. Arzneimittelformulierung nach Anspruch 7, **dadurch gekennzeichnet dass** der Gehalt an Komplexbildner 0,1 bis 50 mg pro 100 ml Lösung beträgt.

9. Arzneimittelformulierung enthaltend als Wirkstoff 1 bis 250 mg pro 100 ml Lösung eine freie Base der Formel **1a'** 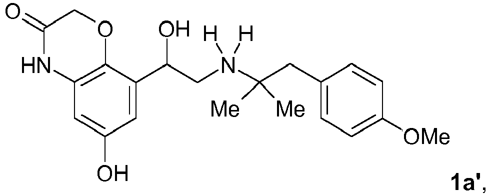 gegebenenfalls in Form ihrer Tautomere, Enantiomere, Mischungen der Enantiomere, Racemate, Solvate oder Hydrate, 1 bis 250 mg pro 100 ml Lösung einen weiteren Wirkstoff **2** ausgewählt aus Tiotropiumsalzen, gegebenenfalls in Form ihrer Tautomere, Enantiomere, Mischungen der Enantiomere, Racemate, Solvate oder Hydrate; wenigstens eine pharmakologisch verträgliche Säure, gegebenenfalls weitere pharmakologisch verträgliche Hilfsstoffe und/oder Komplexbildner sowie als Lösungsmittel Wasser, worin die Arzneimittelformulierung einen pH Wert zwischen 2,5 bis 3,5 hat.

10. Arzneimittelformulierung nach Anspruch 9, worin der Wirkstoff **2** Tiotropiumbromid ist, gegebenenfalls in Form seiner Tautomere, Enantiomere, Mischungen der Enantiomere, Racemate, Solvate oder Hydrate.

11. Verwendung einer Arzneimittelformulierung nach einem der Ansprüche 1 bis 10 zur Herstellung eines Arzneimittels zur Behandlung von Atemwegserkrankungen.

12. Inhalationskit bestehend aus einer Arzneimittelformulierung nach einem der Ansprüche 1 bis 10 und einem zur Vernebelung dieser Arzneimittelformulierung geeigneten Inhalator.

13. Inhalationskit nach Anspruch 12, wobei der Inhalator der Respimat® ist.

## Claims

1. Medicament formulation, containing as active substance a compound of formula **1** 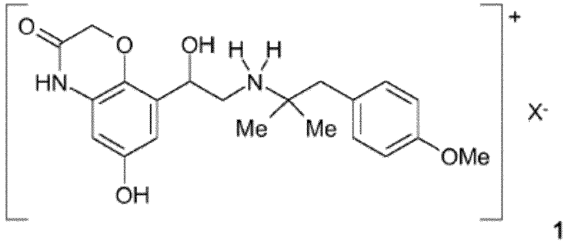 wherein
X- denotes an anion with a single negative charge, preferably an anion with a single negative charge selected from among chloride, bromide, iodide, sulphate, phosphate, methane sulfonate, nitrate, maleate, acetate, benzoate, citrate, salicylate, trifluoroacetate, fumarate, tartrate, oxalate, succinate, benzoate and p-toluene sulfonate;
wherein the medicament formulation according to the invention contains
• 1 to 250 mg free base **1'** per 100 ml of solution,
• 1 to 250 mg free cation of tiotropium salt **2.1'** per 100 ml of solution,
• pure water as solvent,
• at least one pharmacologically acceptable acid,
• optionally further pharmacologically acceptable excipients and/or complexing agents;
wherein the medicament formulation has a pH of 2.5 to 3.5.

2. Medicament formulation according to claim 1, wherein the pharmacologically acceptable acid is selected from the inorganic acids hydrochloric acid, hydrobromic acid, nitric acid, sulphuric acid and phosphoric acid or from the organic acids ascorbic acid, citric acid, malic acid, tartaric acid, maleic acid, succinic acid, fumaric acid, acetic acid, formic acid, propionic acid, sorbic acid, benzoic acid, methanesulfonic acid and benzenesulfonic acid.

3. Medicament formulation according to claim 1 or claim 2 , **characterised in that** it contains benzalkonium chloride as excipient.

4. Medicament formulation according to claim 3, **characterised in that** the content of benzalkonium chloride is 1 to 50 mg per 100 ml of solution.

5. Medicament formulation according to one of claims 1 to 4, **characterised in that** it contains an antioxidant as an additional pharmacologically acceptable excipient.

6. Medicament formulation according to one of claims 1 to 5, **characterised in that** it contains as an additional pharmacologically acceptable excipient an antioxidant selected from among ascorbic acid, propyl gallate, butylhydroxyanisol, butylhydroxytoluene, tert-butylhydroxyquinone and tocopherols.

7. Medicament formulation according to one of claims 1 to 6, **characterised in that** it contains a complexing agent as an additional ingredient.

8. Medicament formulation according to claim 7, **characterised in that** the content of complexing agent is 0.1 to 50 mg per 100 ml of solution.

9. Medicament formulation containing as active substance 1 to 250 mg of a free base of formula **1a'** per 100 ml of solution, 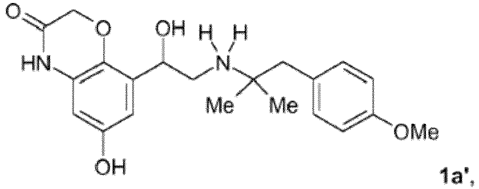 optionally in the form of the tautomers, enantiomers, mixtures of the enantiomers, racemates, solvates or hydrates thereof, 1 to 250 mg per 100 ml of solution of a further active substance **2** selected from among the tiotropium salts, optionally in the form of the tautomers, enantiomers, mixtures of the enantiomers, racemates, solvates or hydrates thereof; at least one pharmacologically acceptable acid, optionally other pharmacologically acceptable excipients and/or complexing agents and, as solvent, water, wherein the medicament formulation has a pH of between 2.5 and 3.5.

10. Medicament formulation according to claim 9, wherein active substance **2** is tiotropium bromide, optionally in the form of the tautomers, enantiomers, mixtures of the enantiomers, racemates, solvates or hydrates thereof.

11. Use of a medicament formulation according to one of claims 1 to 10 for preparing a pharmaceutical composition for the treatment of respiratory diseases.

12. Inhalation kit containing a medicament formulation according to one of claims 1 to 10 and an inhaler suitable for nebulising said medicament formulation.

13. Inhalation kit according to claim 12, wherein the inhaler is a Respimat®.

## Revendications

1. Formulation de médicament, contenant en tant que principe actif un composé de formule 1 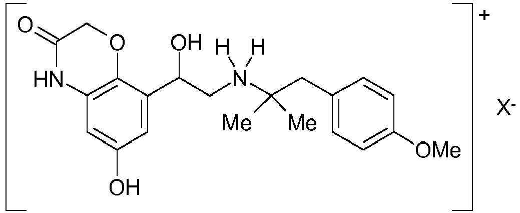 dans laquelle
X- représente un anion chargé simplement négativement, de manière préférée un anion chargé simplement négativement choisi parmi le groupe constitué du chlorure, du bromure, de l'iodure, du sulfate, du méthanesulfonate, du nitrate, du maléate, de l'acétate, du benzoate, du citrate, du salicylate, du trifluoroacétate, du fumarate, du tartrate, de l'oxalate, du succinate, du benzote et du p-toluènesulfonate ;
dans laquelle la formulation de médicament selon l'invention contient
- 1 à 250 mg d'une base libre 1' pour 100 ml de solution,
- 1 à 250 mg du cation libre du sel de tiotropium 2.1' pour 100 ml de solution,
- de l'eau pure en tant que solvant,
- au moins un acide pharmacologiquement acceptable,
- au choix d'autres excipients et/ou agents complexants pharmacologiquement acceptables,
dans laquelle la formulation de médicament présente un pH allant de 2,5 à 3,5.

2. Formulation de médicament selon la revendication 1, dans laquelle l'acide pharmacologiquement acceptable est choisi parmi des acides inorganiques acide chlorhydrique, acide bromhydrique, acide nitrique, acide sulfurique et acide phosphorique ou parmi les acides organiques acide ascorbique, acide citrique, acide malique, acide tartrique, acide maléique, acide succinique, acide fumarique, acide acétique, acide formique, acide propionique, acide sorbique, acide benzoïque, acide méthanesulfonique et acide benzènesulfonique.

3. Formulation de médicament selon l'une quelconque des revendications 1 à 2, **caractérisée en ce qu'**elle contient en tant qu'excipient du chlorure de benzalconium.

4. Formulation de médicament selon la revendication 3, **caractérisée en ce que** la teneur en chlorure de benzalconium est de 1 à 50 mg pour 100 ml de solution.

5. Formulation de médicament selon l'une quelconque des revendications 1 à 4, **caractérisée en ce qu'**elle contient en tant qu'autre excipient pharmacologiquement acceptable un anti-oxydant.

6. Formulation de médicament selon l'une quelconque des revendications 1 à 5, **caractérisée en ce qu'**elle contient en tant qu'autre excipient pharmacologiquement acceptable un anti-oxydant choisi parmi le groupe constitué de l'acide ascorbique, du gallate de propyle, du butylhydroxyanisole, du bultylhydroxytoluène, de la tert-bultylhydroxyquinone, des tocophérols.

7. Formulation de médicament selon l'une quelconque des revendications 1 à 6, **caractérisée en ce qu'**elle contient en tant qu'autre constituant un agent complexant.

8. Formulation de médicament selon la revendication 7, **caractérisé en ce que** la teneur en agent complexant est de 0,1 à 50 mg pour 100 ml de solution.

9. Formulation de médicament contenant en tant que principe actif 1 à 250 mg pour 100 ml de solution d'une base libre de la formule 1a' 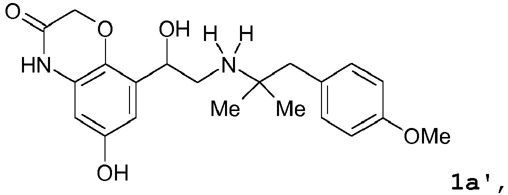 éventuellement sous la forme de ses tautomères, énantiomères, mélanges d'énantiomères, racémates, solvates ou hyrates, 1 à 250 mg pour 100 ml de solution d'un autre principe actif 2 choisi parmi des sels de tiotropium, éventuellement sous la forme de leurs tautomères, énantiomères, mélanges d'énantiomères, racémates, solvates ou hydrates ; au moins
un acide pharmacologiquement acceptable, éventuellement d'autres excipients pharmacologiquement acceptables et/ou des agents complexants ainsi qu'en tant que solvant de l'eau, dans laquelle la formulation de médicament présente une valeur pH comprise entre 2,5 à 3,5.

10. Formulation de médicament selon la revendication 9, dans laquelle le principe actif 2 est du bromure de triotropium, éventuellement sous la forme de ses tautomères, énantiomères, mélanges d'énantiomères, racémates, solvates ou hydrates.

11. Utilisation d'une formulation de médicament selon l'une quelconque des revendications 1 à 10 servant à fabriquer un médicament pour traiter des maladies respiratoires.

12. Kit d'inhalation constitué d'une formulation de médicament selon l'une quelconque des revendications 1 à 10 et d'un inhalateur convenant aux fins de la nébulisation de ladite formulation de médicament.

13. Kit d'inhalation selon la revendication 12, dans lequel l'inhalateur est le Respimat®.
